# EUROPEAN PATENT SPECIFICATION

(11) **EP 2 301 442 B1**
(45) Date of publication and mention of the grant of the patent: **13.02.2013**
(21) Application number: 09820560.2
(22) Date of filing: 09.10.2009
(51) Int. Cl.: A61B 8/12, H04R 17/00, B06B 1/06, H01L 41/083

(54) **ULTRASONIC PROBE**
ULTRASCHALLSONDE
SONDE ULTRASONORE

(30) Priority: 14.10.2008 JP 2008265580; 14.10.2008 JP 2008265581
(43) Date of publication of application: 30.03.2011
(73) Proprietor: Olympus Medical Systems Corp., Tokyo 151-0072 (JP)
(72) Inventor: IMAHASHI Takuya, Tokyo 151-0072 (JP)
(74) Representative: Gunzelmann, Rainer
(86) International application number: PCT/JP2009/067665
(87) International publication number: WO 2010/044382

(56) References cited:
- EP-A1- 1 449 482
- WO-A1-03/024625
- JP-A- 9 084 194
- JP-A- 10 056 690
- JP-A- 10 304 495
- JP-A- 2007 082 629
- US-A- 6 020 675
- US-A1- 2007 038 110

## Description

### Technical Field

The present invention relates to an ultrasound probe provided with a piezocomposite material.

### Background Art

As an apparatus that acquires an ultrasound image of the interior of a living body, an ultrasound probe is known which is provided with an ultrasound transducer unit that transmits/receives ultrasound to/from an insertion portion inserted into the body such as a digestive tract. As one mode of the ultrasound transducer unit, a piezocomposite material is known which is made up of a piezoelectric material such as PZT and resin. The piezocomposite material has a feature of having high coupling coefficient and the acoustic impedance of this material is nearly the same as that of human body.

The piezocomposite material is classified into a plurality of modes according to a connectivity combination of piezoelectric material and resin. For example, as disclosed in Japanese Patent Application Laid-Open Publication No. 62-22634 and Japanese Patent Application Laid-Open Publication No. 6-154208, a piezocomposite material in a mode in which gaps in a plurality of columnar piezoelectric materials having one-dimensional connectivity are filled with resin having three-dimensional connectivity is called a "1-3 type piezocomposite material." Electrodes for giving an electric field to the 1-3 type piezocomposite material is formed on both principal surfaces of the 1-3 type piezocomposite material.

Conventional piezocomposite materials employ a configuration in which wiring is connected to electrodes formed on both principal surfaces, and more particularly, connected to electrodes on piezoelectric materials having high adhesive strength, by wire bonding and the like. When such a wiring connection configuration is employed in an ultrasound probe, wiring is protruded from the principal surfaces of the piezocomposite material, which results in an increase in size of the region including the piezocomposite material in an ultrasound transmitting direction. Such a configuration is not preferable for an ultrasound probe which is configured to be inserted into a body and which requires downsizing.

Document US 6,020,675 discloses an ultrasonic probe comprising a 2-2 type composite piezoelectric element sandwiched between a first electrode and a second electrode The first electrode is shaped as a turning electrode. The extending section of the turning electrode extends perpendicular to an array direction of the layers of the 2-2 type composite piezoelectric element.

Document EP 1 449 482 A1 discloses an ultrasonic probe having a piezoelectric transducer. The piezoelectric transducer comprises a signal electrode and an earth electrode.

Document US 2007/0038110 A1 concerns a motorized ultrasonic scan head capable of rotating an array transducer through 360° of angular rotation in a manner such as to provide acquisition of images in successive scanning planes arranged around the principle axis of the device.

Document WO 03/024625 A1 concerns a method and an apparatus for apodization of transducers.

The present invention has been achieved in view of the above-described circumstances, and an object of the present invention is to provide an ultrasound probe provided with a piezocomposite material and capable of downsizing.

### Disclosure of Invention

### Means for Solving the Problem

An ultrasound probe according to the present invention includes the features of claim 1.

### Brief Description of the Drawings

Fig. 1 is a diagram illustrating a schematic configuration of a distal end portion of an ultrasound probe;
Fig. 2 is a side view of an ultrasound transmitting/receiving section viewed from a direction orthogonal to a plane made up of an insertion axis and a sound axis;
Fig. 3 is a diagram illustrating a III-III cross section of Fig. 2 illustrating a configuration of the 2-2 type piezocomposite material and wiring viewed from the sound axis direction;
Fig. 4 is a diagram illustrating a configuration of a 2-2 type piezocomposite material according to a second embodiment;
Fig. 5 is a side view of an ultrasound transmitting/receiving section according to a third embodiment viewed from a direction orthogonal to a plane defined by an insertion axis and a sound axis;
Fig. 6 is a diagram illustrating a connection configuration of the 2-2 type piezocomposite material and wiring of the third embodiment viewed from a sound axis direction;
Fig. 7 is a VII-VII cross-sectional view of Fig. 6; and
Fig. 8 is a diagram of an ultrasound transmitting/receiving section according to a fourth embodiment viewed from a direction along a sound axis.

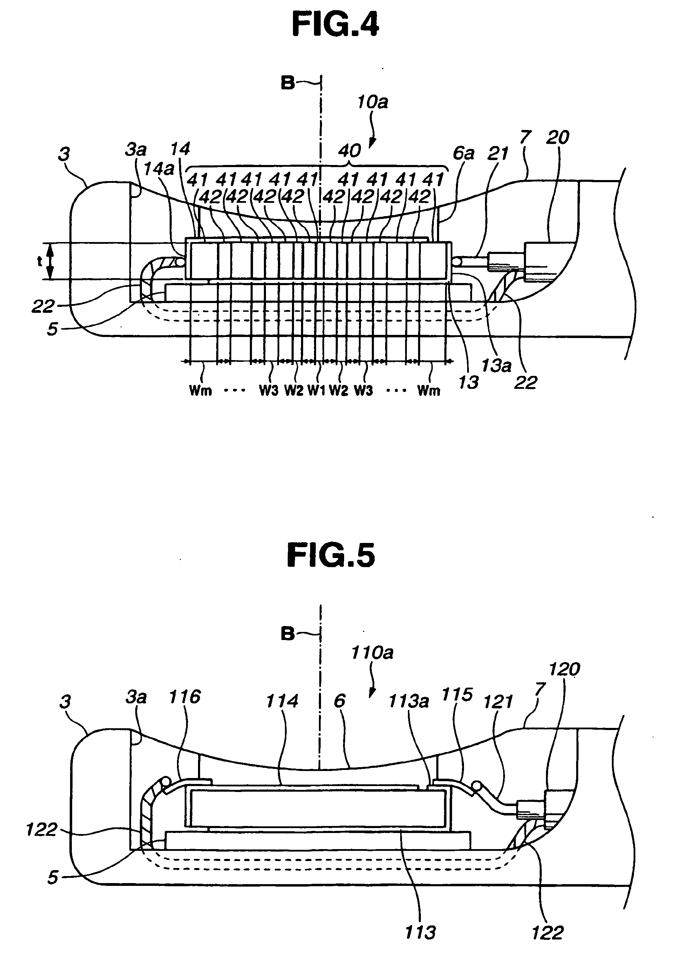

The embodiments of Figs 5-8 do not form part of the invention.

### Best Mode for Carrying Out the Invention

Hereinafter, preferred embodiments of the present invention will be described with reference to the accompanying drawings. In some of the drawings used in the following descriptions, scaling is made to differ from one component to another to illustrate the respective components on the order of size recognizable on the drawings, and the present invention is not limited only to the quantity of components, shapes of components, ratio in size of the components and relative positional relationship between the components described in the drawings.

### (First embodiment)

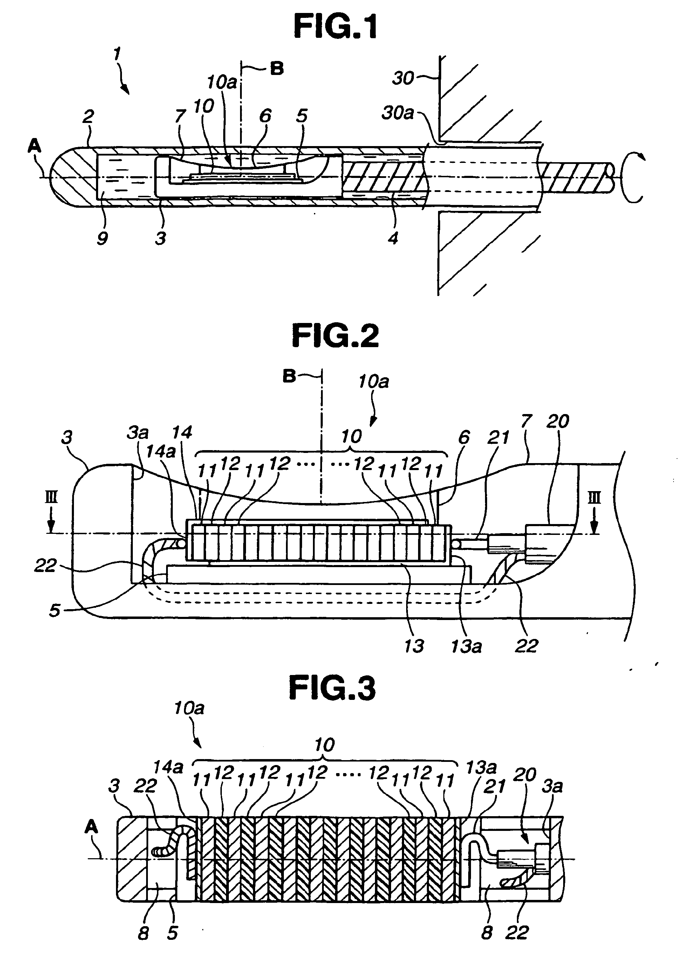

Hereinafter, a first embodiment of the present invention will be described. As shown in Fig. 1, in an ultrasound probe 1 of the present embodiment, at least part thereof is configured more specifically as an insertion portion that can be inserted into a channel pipe 30a provided at the insertion portion of an endoscope 30 inserted into the body of a subject (not shown) and also configured to be insertable/retractable into/from the body of the subject via an opening at a distal end of the channel pipe 30a.

The insertion portion of the ultrasound probe 1 is mainly configured by including a sheath 2 having a cylindrical shape with a closed distal end, a flexible shaft 4 pivotably inserted in the sheath 2 and a housing 3 fixed to a distal end of the flexible shaft 4 to hold an ultrasound transmitting/receiving section 10a, which will be described in detail later. Furthermore, the sheath 2 is filled with an ultrasound transmission medium 9 such as water or oil.

Hereinafter, a central axis of the ultrasound probe 1 along the longitudinal direction of the insertion portion (axis shown by A in Fig. 1) will be referred to as an "insertion axis A."

The flexible shaft 4 is connected to a rotation drive mechanism such as an electric motor provided for an ultrasound observation processor apparatus (not shown) on a proximal end side and a driving force of the rotation drive mechanism to the housing 3 is transmitted by the flexible shaft 4. That is, the housing 3 rotates in the sheath 2 together with the flexible shaft 4 around the insertion axis A as a central axis.

The ultrasound transmitting/receiving section 10a fixed to the housing 3 is configured to be able to transmit an ultrasound beam of a predetermined frequency bandwidth and receive reflected wave of the ultrasound beam from the living body and is electrically connected to the ultrasound observation apparatus via a coaxial cable 20 (not shown in Fig. 1) disposed in the flexible shaft 4.

In the present embodiment, the ultrasound transmitting/receiving section 10a is fixed to the housing 3 so as to transmit the ultrasound beam centered on one predetermined direction on a plane substantially orthogonal to the insertion axis. Hereinafter, the axis that becomes the center of the ultrasound beam transmitted by the ultrasound transmitting/receiving section 10a (axis shown by B in Fig. 1) will be referred to as a "sound axis B."

That is, in the ultrasound probe 1 of the present embodiment, the sound axis B is an axis oriented to a specific direction with respect to the ultrasound transmitting/receiving section 10a and when the housing 3 rotates around the insertion axis A, the sound axis B also rotates around the insertion axis A.

The ultrasound probe 1 whose schematic configuration has been described above is intended to transmit/receive ultrasound while causing the ultrasound transmitting/receiving section 10a to rotate around the insertion axis A and is generally referred to as a "mechanical scanning type ultrasound miniature probe" or the like.

Hereinafter, a detailed configuration of the ultrasound transmitting/receiving section 10a of the ultrasound probe 1 according to the present embodiment will be described.

As shown in Fig. 2 and Fig. 3, the ultrasound transmitting/receiving section 10a is accommodated in an opening 3a formed in side faces of the housing 3. The ultrasound transmitting/receiving section 10a is fixed in the opening 3a with seal resin 7 such that the sound axis B is substantially orthogonal to the insertion axis A.

The ultrasound transmitting/receiving section 10a is configured by including a 2-2 type piezocomposite material 10, a signal electrode 13, a grounding electrode 14, a backing member 5 and an acoustic matching layer 6.

The 2-2 type piezocomposite material 10 is a so-called transducer made up of a plurality of substantially planar piezoelectric layers 11 and a plurality of substantially planar resin layers 12 alternately arrayed. Furthermore, the 2-2 type piezocomposite material 10 according to the present embodiment is configured such that the piezoelectric layers 11 are disposed at both ends in the array direction.

The piezoelectric layers 11 of the 2-2 type piezocomposite material 10 are disposed in a top-to-bottom direction on the sheet when viewed from the front of Fig. 2, that is, disposed so as to effectively produce distortion according to the intensity of the electric field in a direction along the sound axis B. In the following descriptions, a pair of surfaces facing the direction of the electric field that causes distortion in the piezoelectric layer 11 in the rectangular parallelepiped 2-2 type piezocomposite material 10 will be referred to as "principal surfaces."

The 2-2 type piezocomposite material 10 is fixed inside the opening 3a of the housing 3 so that the array directions of the piezoelectric layer 11 and the resin layer 12 are along the insertion axis A.

The material making up the piezoelectric layer 11 is not particularly limited as long as the material is a piezoelectric material that produces distortion according to the electric field or one called an "electrostrictive material" and, for example, piezoelectric ceramics such as PZT can be used.

A pair of electrodes facing each other across the 2-2 type piezocomposite material 10 are disposed on both principal surfaces of the 2-2 type piezocomposite material 10. The pair of electrodes extend on both end faces facing parallel to the insertion axis A of the 2-2 type piezocomposite material 10 respectively.

To be more specific, the pair of electrodes in the present embodiment are made up of the signal electrode 13 to which a signal for giving an electric field to the 2-2 type piezocomposite material 10 is inputted and the grounding electrode 14 which is set to a grounding potential. The signal electrode 13 and the grounding electrode 14 are, for example, thin-films made of a conductive material such as metal like gold or copper and formed using a publicly known technique such as a physical vapor deposition method or a chemical vapor deposition method. The piezoelectric material is known to have high adhesive strength to the electrode compared to resin as disclosed in Japanese Patent Application Laid-Open Publication No. 62-22634.

The signal electrode 13 is formed on the principal surface (lower surface in Fig. 2) opposite to the ultrasound transmitting direction of the 2-2 type piezocomposite material 10. Furthermore, the signal electrode 13 has an extending section 13a that extends so as to cover an end face facing the proximal end side of the 2-2 type piezocomposite material 10 from the principal surface of the 2-2 type piezocomposite material 10.

On the other hand, the grounding electrode 14 is formed on the principal surface (upper surface in Fig. 2) facing the ultrasound transmitting direction of the 2-2 type piezocomposite material 10. Furthermore, the grounding electrode 14 has an extending section 14a that extends so as to cover the end face facing the distal end side of the 2-2 type piezocomposite material 10 from the principal surface of the 2-2 type piezocomposite material 10.

Here, since the piezoelectric layers 11 are disposed at both ends in the array direction of the 2-2 type piezocomposite material 10 as described above, the extending section 13a of the signal electrode 13 and the extending section 14a of the grounding electrode 14 are formed on the end faces of the piezoelectric layers 11 disposed at both ends in the array direction respectively.

The signal electrode 13 and the grounding electrode 14 may also be configured to be formed on the principal surfaces opposite to those of the present embodiment respectively. Furthermore, the extending section 13a of the signal electrode 13 and the extending section 14a of the grounding electrode 14 may also be formed on the end face of the 2-2 type piezocomposite material 10 opposite to that of the present embodiment.

The backing member 5 is disposed on the surface opposite to the 2-2 type piezocomposite material 10 of the signal electrode 13. The backing member 5 is made of resin or the like and intended to attenuate unnecessary ultrasound generated from the 2-2 type piezocomposite material 10.

On the other hand, the acoustic matching layer 6 for performing acoustic matching between the 2-2 type piezocomposite material 10 and the ultrasound transmission medium 9 is disposed on the surface opposite to the 2-2 type piezocomposite material 10 of the grounding electrode 14. Having a concave surface opposite to the grounding electrode 14, the acoustic matching layer 6 also functions as an acoustic lens that causes ultrasound generated by the 2-2 type piezocomposite material 10 to converge and forms an ultrasound beam along the sound axis B.

The acoustic matching layer 6 may also be made up of the layer that performs acoustic matching and the layer that functions as an acoustic lens as different members.

Next, the configuration in which the ultrasound transmitting/receiving section 10a and wiring are connected will be described in detail.

The coaxial cable 20 is configured by including a signal wiring 21 disposed in the center and a grounding wiring 22 that covers the perimeter of the signal wiring 21 via an insulator. As described above, the coaxial cable 20 is inserted in the flexible shaft 4 and a distal end thereof extends out into the opening 3a of the housing 3.

The signal wiring 21 and the grounding wiring 22 may also be configured so as to be individually inserted into the flexible shaft 4 and extend out into the housing 3 instead of making up the coaxial cable 20.

A distal end of the signal wiring 21 is connected to the extending section 13a of the signal electrode 13 formed on the 2-2 type piezocomposite material 10. Furthermore, in the present embodiment, the signal wiring 21 is connected to the extending section 13a of the signal electrode 13 so that the distal end thereof extends in a direction substantially orthogonal to the array direction of the 2-2 type piezocomposite material 10 (direction substantially orthogonal to the insertion axis A).

By connecting the signal wiring 21 and the extending section 13a with the signal wiring 21 placed along the extending section 13a in this way, it is possible to increase the connection length using solder, conductive adhesive or the like without increasing the height of the ultrasound transmitting/receiving section 10a in the sound axis B direction, improve reliability of the connection between the signal wiring 21 and the signal electrode 13 and make the signal wiring thinner.

On the other hand, the distal end of the grounding wiring 22 is connected to the extending section 14a of the grounding electrode 14 formed on the 2-2 type piezocomposite material 10. To be more specific, the grounding wiring 22 extends to the distal end side of the 2-2 type piezocomposite material 10 so as to wrap around the side of the backing member 5 opposite to the 2-2 type piezocomposite material 10 in the housing 3 and is connected to the extending section 14a of the grounding electrode 14 formed on the 2-2 type piezocomposite material 10 using solder, conductive adhesive or the like. The region where the grounding wiring 22 wraps around the backing member 5 is fixed by seal resin 8 as shown in Fig. 3.

Furthermore, in the present embodiment, the grounding wiring 22 is connected to the extending section 14a of the grounding electrode 14 so that the distal end thereof extends in a direction substantially orthogonal to the array direction of the 2-2 type piezocomposite material 10 (direction substantially orthogonal to the insertion axis A).

By connecting the grounding wiring 22 and the extending section 14a with the grounding wiring 22 placed along the extending section 14a in this way, it is possible to improve reliability of the connection between the grounding wiring 22 and the grounding electrode 14 as in the case of the signal wiring 21.

As described above, the ultrasound transmitting/receiving section 10a is electrically connected to an ultrasound observation apparatus via the signal wiring 21 and the grounding wiring 22.

When the extending section 13a of the signal electrode 13 is formed so as to cover the end face on the distal end side of the 2-2 type piezocomposite material 10, it goes without saying that the signal wiring 21 extends to the distal end side as opposed to the present embodiment and is connected to the extending section 13a of the signal electrode 13 formed on the end face on the distal end side.

In the ultrasound probe 1 configured as described above, the piezoelectric layers 11 are disposed at both ends of the 2-2 type piezocomposite material 10 in the array direction and the signal electrode 13 and the grounding electrode 14 are formed by extending so as to cover the end face facing the outside in the array direction of the piezoelectric layers 11 at both ends. The signal wiring 21 and the grounding wiring 22 are connected to the extending sections 13a and 14a of the signal electrode 13 and the grounding electrode 14 formed on the end face of the 2-2 type piezocomposite material 10 respectively.

In other words, in the present embodiment, the wiring connected to the electrodes formed on the 2-2 type piezocomposite material 10 is connected to both end faces of the 2-2 type piezocomposite material 10 in the array direction. Thus, the ultrasound probe 1 of the present embodiment can realize downsizing in the sound axis B direction compared to the conventional configuration in which wiring is connected to the principal surface of the piezoelectric material. That is, according to the present embodiment, it is possible to make the ultrasound probe 1 in a smaller diameter than in the related art.

Furthermore, the signal wiring 21 and the grounding wiring 22 of the present embodiment are connected to the extending section 13a of the signal electrode 13 and the extending section 14a of the grounding electrode 14 formed on planar end faces having relatively large areas. Here, in the 2-2 type piezocomposite material 10, coupling strength (Adhesive, Coulomb, etc.) between the piezoelectric layer 11 and the electrode is higher than that between the resin layer 12 and the electrode.

That is, the ultrasound probe 1 of the present embodiment has higher adhesive strength between the piezocomposite material and the electrode compared to the configuration of the conventional composite type piezoelectric material in which electrodes are formed on the principal surface made up of the piezoelectric layer and the resin layer, and can thereby prevent an operation failure caused by the electrode peeling off the piezocomposite material.

### (Second embodiment)

Hereinafter, a second embodiment of the present invention will be described. The second embodiment is different from the aforementioned first embodiment only in the configurations of the 2-2 type piezocomposite material and the acoustic matching layer. Therefore, hereinafter only the difference will be described and components similar to those in the first embodiment will be assigned the same reference numerals and descriptions thereof will be omitted as appropriate.

As shown in Fig. 4, a 2-2 type piezocomposite material 40 of the present embodiment is made up of a plurality of piezoelectric layers 41 and a plurality of substantially planar resin layers 42 alternately arrayed as in the case of the first embodiment and the piezoelectric layers 41 are disposed at both ends in the array direction.

Here, in the present embodiment, the dimension of a width W of the piezoelectric layer 41 making up the 2-2 type piezocomposite material 40 (size in the array direction) is set so as to continuously vary to be the largest at both ends in the array direction and the smallest in the center in the array direction.

To be more specific, as shown in Fig. 4, when the width W of the piezoelectric layer 41 from the piezoelectric layer 41 located in the center of the 2-2 type piezocomposite material 40 in the array direction toward the ends in the array direction is defined in order as W1, W2, ... Wn (n is a natural number and satisfies 1≤n≤m), the relationship between Wn's is W1<W2<W3< ... <Wm.

Furthermore, widths of all resin layers 42 have the same fixed value. Furthermore, a thickness t of the 2-2 piezocomposite material 40 is constant throughout the unit.

The 2-2 type piezocomposite material 40 of the present embodiment shown in Fig. 4 is configured by including an odd number of piezoelectric layers 41, and therefore there is only pne piezoelectric layer 41 of width W1 located in the center, but if the 2-2 type piezocomposite material 40 is made up of an even number of piezoelectric layers 41, there are two piezoelectric layers 41 of width W1 on both sides of the sound axis B.

In the 2-2 type piezocomposite material 40 of the aforementioned present embodiment, the width W of the piezoelectric layer 41 continuously varies in the array direction, and therefore the resonance frequency differs from one piezoelectric material to another and the 2-2 type piezocomposite material 40 is made up of piezoelectric layers 41 having a plurality of different vibration modes. Therefore, the ultrasound probe 1 of the present embodiment can transmit/receive ultrasound in a broader frequency bandwidth than the first embodiment.

Furthermore, an acoustic matching layer 6a of the present embodiment is configured so as to have a thickness which is continuously variable according to a variation in the width W of the piezoelectric layer 41 making up the 2-2 type piezocomposite material 40. To be more specific, the thickness of the acoustic matching layer 6a is set to a value appropriate for a vibration mode of the piezoelectric layer 41 disposed directly therebelow and the acoustic matching layer 6a of the present embodiment has such a shape that the thickness thereof is largest at both ends and smallest in the center in the array direction of the 2-2 type piezocomposite material 40.

That is, the acoustic matching layer 6a of the present embodiment realizes excellent acoustic matching between the 2-2 type piezocomposite material 40 having a broad frequency bandwidth and the living body and also has a function as an acoustic lens that causes ultrasound to converge. Thus, according to the present embodiment, it is possible to acquire an ultrasound image of higher image quality by the ultrasound probe 1.

### (Third embodiment, not forming part of the invention)

Incidentally, in the case of a piezocomposite material applicable to an ultrasound probe designed to be inserted in the body, individual piezoelectric materials are finely structured, and it is thereby difficult to position and connect wiring on electrodes formed on the piezoelectric materials, and moreover, the connection strength degrades.

Furthermore, when wiring is connected to the principal surface of a fine piezoelectric material by soldering or the like, the electromechanical conversion efficiency degrades due to influences of a weight load of the solder. In the case of a piezoelectric material of reduced size and thickness such as an ultrasound probe in particular, a mass ratio of the solder to the mass of the piezoelectric material is large, which may lead to characteristic deterioration. Furthermore, when the thickness of the solder exceeds a predetermined thickness, an unnecessary vibration mode appears and the image quality of an ultrasound image may thereby degrade.

Thus, with the ultrasound probe provided with a piezocomposite material, it is expected to improve reliability of a wiring connection to the piezocomposite material and reduce influences of the wiring connection on characteristics of the piezocomposite material.

An ultrasound probe according to a third embodiment, which will be described below, is an ultrasound probe provided with a piezocomposite material that realizes improvement of reliability of the wiring connection to the piezocomposite material and reduction of influences of the wiring connection on characteristics of the piezocomposite material. In the following descriptions, components similar to those in the first embodiment will be assigned the same reference numerals and descriptions thereof will be omitted as appropriate.

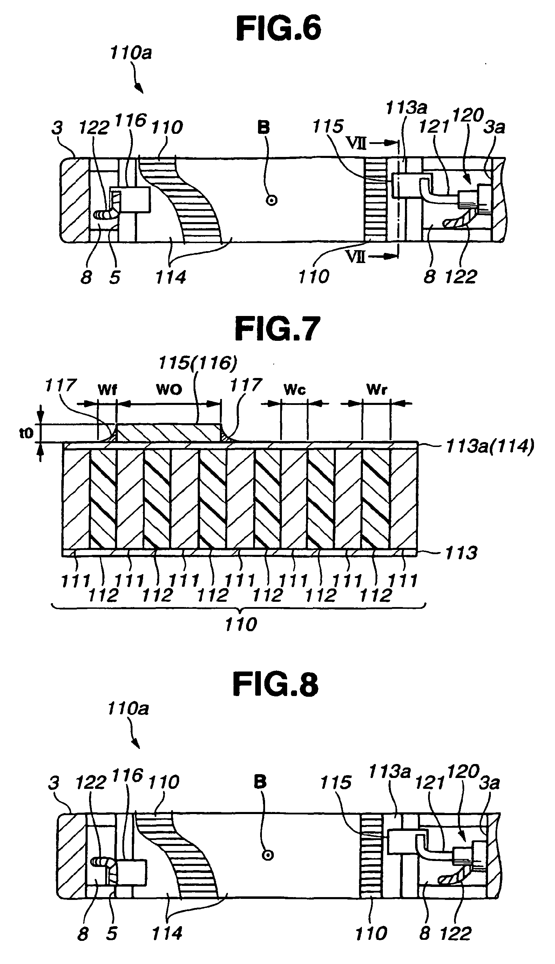

As shown in Fig. 5 and Fig. 6, an ultrasound transmitting/receiving section 110a, which is the ultrasound probe of the present embodiment, is accommodated in an opening 3a formed in side faces of the housing 3. The ultrasound transmitting/receiving section 110a is fixed in the opening 3a with seal resin 7 so that the sound axis B is substantially orthogonal to the insertion axis A.

The ultrasound transmitting/receiving section 110a is configured by including a 2-2 type piezocomposite material 110, a signal electrode 113, a grounding electrode 114, the backing member 5 and the acoustic matching layer 6.

As shown in Fig. 7, the 2-2 type piezocomposite material 110 is a so-called electromechanical conversion element made up of a plurality of substantially planar piezoelectric layers 111 and a plurality of substantially planar resin layers 112 alternately arrayed. In the present embodiment, all of the plurality of piezoelectric layers 111 have a width Wc and all of the plurality of resin layers 112 have a width Wr. Here, the width Wc of the piezoelectric layer 111 and the width Wr of the resin layer 112 refer to outside dimensions of the piezoelectric layer 111 and the resin layer 112 in the array direction.

The piezoelectric layer 111 of the 2-2 type piezocomposite material 110 is disposed so as to effectively produce distortion according to the strength of the electric field in an upward and downward direction on the surface of the sheet when viewed from the front of Fig. 5, that is, in a direction along the sound axis B. In the following descriptions, in the rectangular parallelepiped 2-2 type piezocomposite material 110, a pair of surfaces facing the direction of the electric field that produces distortion in the piezoelectric layer 111 is referred to as "principal surfaces."

As shown in Fig. 6, the 2-2 type piezocomposite material 110 is fixed in the opening 3a of the housing 3 so that the array directions of the piezoelectric layers 111 and the resin layers 112 are substantially orthogonal to the insertion axis A when viewed from the direction along the sound axis B.

The material making up the piezoelectric layers 111 is not particularly limited as long as the material is a piezoelectric material that produces distortion according to the electric field or one called an "electrostrictive material" and, for example, piezoelectric ceramics such as PZT can be used.

A pair of electrodes mutually facing across the 2-2 type piezocomposite material 110 are disposed on both principal surfaces of the 2-2 type piezocomposite material 110. To be more specific, the pair of electrodes according to the present embodiment are made up of the signal electrode 113 to which a signal for giving an electric field to the 2-2 type piezocomposite material 110 is inputted and the grounding electrode 114 which is set to a grounding potential. The signal electrode 113 and the grounding electrode 114 are, for example, a thin-film made of a conductive material such as metal and formed using a publicly known technique such as a physical vapor deposition method or a chemical vapor deposition method.

The signal electrode 113 is formed on the principal surface (lower surface in Fig. 5) opposite to an ultrasound transmitting direction of the 2-2 type piezocomposite material 110. Furthermore, the signal electrode 113 has an extending section 113a that wraps around on the end face on the proximal end side of the 2-2 type piezocomposite material 110 and covers part of the principal surface (upper surface in Fig. 5) facing the ultrasound transmitting direction of the 2-2 type piezocomposite material 110.

On the other hand, the grounding electrode 114 is formed on the principal surface facing the ultrasound transmitting direction of the 2-2 type piezocomposite material 110. The grounding electrode 114 is separated by a predetermined width from the extending section 113a of the signal electrode 113 formed on the same principal surface of the 2-2 type piezocomposite material 110.

That is, in the present embodiment, when the 2-2 type piezocomposite material 110 is viewed from a direction along the sound axis B (viewpoint in Fig. 6), the extending section 113a of the signal electrode 113 and the grounding electrode 114 are formed on the principal surface of the 2-2 type piezocomposite material 110.

The signal electrode 113 and the grounding electrode 114 may also be configured to be formed on principal surfaces opposite to those of the present embodiment respectively. In this case, part of the grounding electrode 114 formed on the principal surface opposite to the ultrasound transmitting side of the 2-2 type piezocomposite material 110 wraps around on the end face on the proximal end side of the 2-2 type piezocomposite material 110 and extends on the principal surface facing the ultrasound transmitting direction of the 2-2 type piezocomposite material 110.

The backing member 5 is disposed on the surface opposite to the 2-2 type piezocomposite material 110 of the signal electrode 113. The backing member 5 is made of resin or the like and intended to attenuate unnecessary ultrasound generated from the 2-2 type piezocomposite material 110.

On the other hand, the acoustic matching layer 6 for performing acoustic matching between the 2-2 type piezocomposite material 110 and the ultrasound transmission medium 9 is disposed on the surface opposite to the 2-2 type piezocomposite material 110 of the grounding electrode 114. The acoustic matching layer 6 has a concave-shaped surface opposite to the grounding electrode 114 and also functions as an acoustic lens that causes ultrasound generated by the 2-2 type piezocomposite material 110 to converge and form an ultrasound beam along the sound axis B.

For the acoustic matching layer 6, the layer for performing acoustic matching and the layer functioning as an acoustic lens may be made up of different members.

Next, the configuration in which the ultrasound transmitting/receiving section 110a and wiring are connected will be described in detail.

A coaxial cable 120 is configured by including signal wiring 121 inserted in the center and a grounding wiring 122 that covers the perimeter of the signal wiring 121 via an insulator. As described above, the coaxial cable 120 is inserted into the flexible shaft 4 and a distal end thereof extends out into the opening 3a of the housing 3.

The signal wiring 121 and the grounding wiring 122 may also be configured to be individually inserted into the flexible shaft 4 and extend out into the housing 3 instead of making up the coaxial cable 120.

The distal end of the signal wiring 121 is connected to the extending section 113a of the signal electrode 113 formed on the principal surface of the 2-2 type piezocomposite material 110 via a conductive member 115. The conductive member 115 is made of a material, at least the surface of which is conductive, and has a shape, which will be described in detail later.

On the other hand, the distal end of the grounding wiring 122 is connected to the grounding electrode 114 formed on the 2-2 type piezocomposite material 110 via a conductive member 116. More specifically, the grounding wiring 122 extends on the distal end side of the 2-2 type piezocomposite material 110 in the housing 3 so as to wrap around the side of the backing member 5 opposite to the 2-2 type piezocomposite material 110 and is connected to the conductive member 116 connected to the front edge of the grounding electrode 114. The region of the grounding wiring 122 that wraps around the backing member 5 is fixed with the seal resin 8 as shown in Fig. 6.

The method for connecting the conductive members 115 and 116, the extending section 113a of the signal electrode 113 and the grounding electrode 114 and the method for connecting the conductive members 115 and 116 and the signal wiring 121 and the grounding wiring 122 are not particularly limited as long as the methods are bonding methods having conductivity such as eutectic bonding, diffusion bonding, conductive adhesive or the like, and bonding is performed by soldering as an example in the present embodiment.

As shown in Fig. 7, the conductive members 115 and 116 have a flat cross section having a width W0 and a thickness t0 To be more specific, the width W0 of the conductive member 115 is greater than a width of an array of two piezoelectric layers 111 and one resin layer 112 and the thickness t0 is smaller than a combined width of one piezoelectric layer 111 and one resin layer 112. That is, relationships of W0>Wc+Wr+Wc and t0<Wc+Wr are satisfied.

The present embodiment is configured such that the signal wiring 121 and the grounding wiring 122 are connected to the 2-2 piezocomposite material 110 via the conductive members 115 and 116, but it goes without saying that if the cross-sectional shapes of the distal ends of the signal wiring 121 and the grounding wiring 122 satisfy conditions similar to the conditions of the cross-sectional shapes of the conductive members 115 and 116, there can also be a configuration in which the signal wiring 121 and the grounding wiring 122 are directly connected to the 2-2 type piezocomposite material 110.

When the conductive members 115 and 116 having the cross-sectional shapes that satisfy the aforementioned conditions are connected to the extending section 113a of the signal electrode 113 and the grounding electrode 114 formed on the principal surface of the 2-2 type piezocomposite material 110, the conductive members 115 and 116 are always connected to the extending section 113a and the grounding electrode 114 on the principal surface of two or more piezoelectric layers 111 irrespective of the positions thereof.

For this reason, positioning of the conductive members 115 and 116 with respect to the 2-2 type piezocomposite material 110 becomes easier compared to the conventional configuration in which wiring is connected to the electrodes on the individual piezoelectric layers. Furthermore, even if peeling or the like occurs on the principal surface of one piezoelectric layer 111, since the conductive members 115 and 116 are connected on the principal surface of another piezoelectric layer 111, continuity between the conductive members 115 and 116 and the extending section 113a and the grounding electrode 114 is never lost.

That is, according to the present embodiment, connection between the 2-2 type piezocomposite material 110 and wiring becomes easier and the connection strength improves, and it is thereby possible to improve reliability of the connection between the 2-2 type piezocomposite material 110 and wiring.

Furthermore, since the thickness t0 of the conductive member 115 or 116 is made smaller than the combined width of one piezoelectric layer 111 and one resin layer 112 in the present embodiment, it is possible to regulate the thickness (substantially identical to t0) of a solder fillet 117 formed around the conductive member 115 or 116 as shown in Fig. 7 so as to be smaller than 1/2 wavelength of ultrasound generated by the 2-2 type piezocomposite material 110. This can suppress appearance of any unnecessary vibration mode due to influences of solder.

Furthermore, according to the present embodiment, it is possible to also regulate the width of a wet area Wf of the solder fillet 117 by the thickness t0 of the conductive member 115 or 116, and thereby reduce the mass of solder existing on the principal surface of one piezoelectric layer 111 and prevent the electromechanical conversion efficiency of the 2-2 type piezocomposite material 110 from lowering due to influences of the mass of solder.

As described above, according to the present embodiment, it is possible to improve reliability of a wiring connection to the 2-2 piezocomposite material 110 in the ultrasound probe 1 and suppress influences of the wiring connection on characteristics of the 2-2 piezocomposite material 110.

In the aforementioned embodiment, the conductive members 115 and 116 are connected to the signal electrode 113 and the grounding electrode 114 on the principal surface of the 2-2 type piezocomposite material 110, but the conductive members 115 and 116 may also be configured to be connected to the signal electrode 113 and the grounding electrode 114 on both end faces in a direction along the insertion axis of the 2-2 type piezocomposite material 110.

### (Fourth embodiment, not forming part of the invention)

Hereinafter, a fourth embodiment of the present invention will be described. The fourth embodiment is different from the aforementioned third embodiment only in positions in which the conductive members 115 and 116 are connected to the 2-2 type piezocomposite material. Therefore, only the difference will be described below and components similar to those of the third embodiment will be assigned the same reference numerals and descriptions thereof will be omitted as appropriate.

As shown in Fig. 8, the conductive members 115 and 116 in the present embodiment are connected to the extending section 113a of the signal electrode 113 and the grounding electrode 114 at positions not overlapping each other in a direction along the insertion axis.

That is, of a plurality of piezoelectric layers 111 making up the 2-2 type piezocomposite material 110 in the present embodiment, the conductive members 115 and 116 are never connected on the principal surface of the same piezoelectric layer 111.

Thus, it is possible to make the mass of solder on the principal surface of one piezoelectric layer 111 smaller than that in the third embodiment and more effectively prevent the electromechanical conversion efficiency of the 2-2 type piezocomposite material 110 from lowering due to influences of the mass of solder.

## Claims

1. An ultrasound probe (1) comprising:
a 2-2 type piezocomposite material (10) made up of piezoelectric layers (11) and resin layers (12) alternately arrayed, the piezoelectric layers (11) being arranged at both ends in an array direction;
a signal electrode (13) disposed on one principal surface of the 2-2 type piezocomposite material (10);
a grounding electrode (14) disposed on the other principal surface of the 2-2 type piezocomposite material (10);
signal wiring (21); and
grounding wiring (22),
**characterized in that**
the signal electrode (13) comprises an extending section (13a) that extends on one end face in the array direction of the 2-2 type piezocomposite material (10),
the grounding electrode (14) comprises an extending section (14a) that extends on the other end face in the array direction of the 2-2 type piezocomposite material (10),
the signal wiring (21) is connected to the extending section (13a) of the signal electrode (13), and
the grounding wiring (22) is connected to the extending section (14a) of the grounding electrode (14).

2. The ultrasound probe (1) according to claim 1, wherein the ultrasound probe (1) is configured to be inserted into a pipe disposed in an endoscope and inserted into a body of a subject, and
the 2-2 type piezocomposite material (10) is disposed so that the array direction is along an insertion axis of the ultrasound probe (1).

3. The ultrasound probe (1) according to claim 1, wherein the 2-2 type piezocomposite material (10) comprises at least three of the piezoelectric layers (11),
the piezoelectric layers (11) have at least two types of widths,
the widest piezoelectric layers (11) are disposed at both ends in the array direction and the narrowest piezoelectric layer (11) is disposed in a center in the array direction.

4. The ultrasound probe (1) according to claim 3, wherein the widths of the piezoelectric layers (11) continuously vary with respect to the array direction.

## Patentansprüche

1. Ultraschallsonde (1) mit:
einem 2-2-Piezokompositmaterial (10), das aus abwechselnd angeordneten piezoelektrischen Schichten (11) und Harzschichten (12) besteht, wobei die piezoelektrischen Schichten (11) an beiden Enden in einer Anordnungsrichtung angeordnet sind;
einer Signalelektrode (13), die auf einer Hauptfläche des 2-2-Piezokompositmaterials (10) angeordnet ist;
einer Erdungselektrode (14), die auf der anderen Hauptfläche des 2-2-Piezokompositmaterials (10) angeordnet ist;
einer Signalverdrahtung (21); und
einer Erdungsverdrahtung (22),
**dadurch gekennzeichnet, dass**
die Signalelektrode (13) einen Verlängerungsabschnitt (13a) umfasst, der sich an einer Endfläche in der Anordnungsrichtung des 2-2-Piezokompositmaterials (10) erstreckt,
die Erdungselektrode (14) einen Verlängerungsabschnitt (14a) umfasst, der sich an der anderen Endfläche in der Anordnungsrichtung des 2-2-Piezokompositmaterials (10) erstreckt,
die Signalverdrahtung (21) mit dem Verlängerungsabschnitt (13a) der Signalelektrode (13) verbunden ist, und
die Erdungsverdrahtung (22) mit dem Verlängerungsabschnitt (14a) der Erdungselektrode (14) verbunden ist.

2. Ultraschallsonde (1) nach Anspruch 1, wobei die Ultraschallsonde (1) dazu ausgebildet ist, in ein Rohr eingeführt zu werden, das in einem Endoskop angeordnet ist und in einen Körper eines Patienten eingeführt wird, und
das 2-2-Piezokompositmaterial (10) so angeordnet ist, dass die Anordnungsrichtung entlang einer Einführachse der Ultraschallsonde (1) verläuft.

3. Ultraschallsonde (1) nach Anspruch 1, wobei das 2-2-Piezokompositmaterial (10) mindestens drei der piezoelektrischen Schichten (11) umfasst,
die piezoelektrischen Schichten (11) mindestens zwei Arten von Breiten aufweisen,
die breitesten piezoelektrischen Schichten (11) an beiden Enden in der Anordnungsrichtung angeordnet sind und die schmalste piezoelektrische Schicht (11) in einer Mitte in der Anordnungsrichtung angeordnet ist.

4. Ultraschallsonde (1) nach Anspruch 3, wobei die Breiten der piezoelektrischen Schichten (11) fortlaufend in Bezug auf die Anordnungsrichtung variieren.

## Revendications

1. Sonde ultrasonore (1) comprenant :
un matériau piézocomposite de type 2-2 (10) constitué de couches piézoélectriques (11) et de couches de résine (12) alternées en réseau, les couches piézoélectriques (11) étant agencées aux deux extrémités dans un sens de réseau ;
une électrode de signal (13) disposée sur une surface principale du matériau piézocomposite de type 2-2 (10) ;
une électrode de terre (14) disposée sur l'autre surface principale du matériau piézocomposite de type 2-2 (10) ;
un câblage de signaux (21) ; et
un câblage de terre (22),
**caractérisée en ce que**
l'électrode de signal (13) comprend une section d'extension (13a) qui s'étend sur une face d'extrémité dans le sens de réseau du matériau piézocomposite de type 2-2 (10),
l'électrode de terre (14) comprend une section d'extension (14a) qui s'étend sur l'autre face d'extrémité dans le sens de réseau du matériau piézocomposite de type 2-2 (10),
le câblage de signaux (21) est connecté à la section d'extension (13a) de l'électrode de signal (13), et
le câblage de terre (22) est connecté à la section d'extension (14a) de l'électrode de terre (14).

2. Sonde ultrasonore (1) selon la revendication 1, dans laquelle la sonde ultrasonore (1) est configurée pour être insérée dans un tuyau disposé dans un endoscope et inséré dans un corps d'un sujet, et
le matériau piézocomposite de type 2-2 (10) est disposé de telle manière que le sens de réseau est le long d'un axe d'insertion de la sonde ultrasonore (1).

3. Sonde ultrasonore (1) selon la revendication 1, dans laquelle
le matériau piézocomposite de type 2-2 (10) comprend au moins trois des couches piézoélectriques (11),
les couches piézoélectriques (11) ont au moins deux types de largeurs,
les couches piézoélectriques (11) les plus larges sont disposées aux deux extrémités dans le sens de réseau et la couche piézoélectrique (11) la plus étroite est disposée dans un centre dans le sens de réseau.

4. Sonde ultrasonore (1) selon la revendication 3, dans laquelle les largeurs des couches piézoélectriques (11) varient de façon continue par rapport au sens de réseau.
